# EUROPÄISCHE PATENTSCHRIFT

(11) **EP 0 426 717 B1**
(45) Veröffentlichungstag und Bekanntmachung des Hinweises auf die Patenterteilung: **24.04.1996**
(21) Anmeldenummer: 89908407.3
(22) Anmeldetag: 24.07.1989
(51) Int. Cl.: C07D 471/04, G11C 13/04, G11B 7/24

(54) **OPTISCHE DATENSPEICHER**
OPTICAL MEMORY
MEMOIRE OPTIQUE

(30) Priorität: 29.07.1988 DE 3825943; 24.01.1989 DE 3901988; 14.03.1989 DE 3908312
(43) Veröffentlichungstag der Anmeldung: 15.05.1991
(73) Patentinhaber: RIEDEL-DE HAEN AKTIENGESELLSCHAFT, D-30926 Seelze (DE)
(72) Erfinder: LANGHALS, Heinz, D-8012 Ottobrunn (DE); POTRAWA, Thomas, D-8015 Markt Schwaben (DE)
(74) Vertreter: Muley, Ralf, Dr.
(86) Internationale Anmeldenummer: EP8900866
(87) Internationale Veröffentlichungsnummer: WO9001480

(56) Entgegenhaltungen:
- EP-A- 0 133 156
- EP-A- 0 159 397
- EP-A- 0 181 290
- EP-A- 0 217 214
- DE-A- 2 153 775
- DE-A- 2 210 050
- DE-A- 2 446 700
- US-A- 3 668 663
- US-A- 3 683 336
- US-A- 3 720 921
- CHEM. BER., 120(1987), 1075;
- ANGEW. CHEM., 99(1987), 566.

## Beschreibung

Die vorliegende Erfindung betrifft die Verwendung von Farbstoffen mit mindestens zwei verschieden gefärbten Modifikationen als Speichermedien in optischen Datenspeichern. Weiterhin betrifft die Erfindung optische Datenspeicher, die als Speichermedium einen Farbstoff mit mindestens zwei verschieden gefärbten Modifikationen enthält, und ein Verfahren zum Einlesen, Auslesen und Löschen dieser Datenspeicher sowie besonders geeignete Farbstoffe für die Verwendung in optischen Datenspeichern und Verfahren zur Herstellung dieser Farbstoffe.

Bei der Datenverarbeitung, z.B. in Computersystemen, aber auch in anderen Bereichen, wie z.B. im Audio- und Videobereich, werden Datenspeicher für die Speicherung und Verarbeitung von Daten bzw. Signalen benötigt. Hierfür werden bisher überwiegend magnetische Speichersysteme verwendet. Bei ihnen läßt sich jedoch eine Erhöhung der Speicherdichte nur schwierig erreichen. Außerdem sind sie gegenüber magnetischen Feldern empfindlich und die Langzeitstabilität der gespeicherten Daten ist problematisch.

Es sind auch bereits optische Datenspeicher, wie z.B. CD-Platten für die Speicherung von Audiosignalen, bekannt, bei denen das Signal mit einem Laserstrahl ausgelesen wird (siehe z.B. DE-A 21 53 775). Derartige Systeme besitzen eine hervorragende Langzeitstabilität. Die einmal eingeschriebenen Daten bzw. Signale können viele Male ausgelesen werden.

Bei diesen Systemen wird zum Erreichen einer hohen Informationsdichte eine Grenzfläche oder eine Grenzschicht, z.B. eine Metallschicht, mit einem Laserstrahl abgetastet und eingeschriebene Veränderungen der Grenzfläche als Veränderungen der Reflexion oder der Transmission des Laserstrahls registriert. Die Information wird dabei entweder thermisch, wie etwa durch Aufschmelzen der Grenzschicht, oder photochemisch, so z.B. durch Ausbleichprozesse von Farbstoffen, beschrieben.

Diese Systeme haben jedoch den Nachteil, daß sie nicht vom Anwender beschrieben und auch nicht gelöscht werden können. Es sind auch löschbare optische Speicher entwickelt worden, die auf magneto-optischer Basis oder auf der Phasenumwandlung von Legierungen beruhen. Derartige optische Speicher haben jedoch wieder den Nachteil einer mangelnden Langzeitstabilität. Außerdem werden für den Lese-und Schreibvorgang zum Teil aufwendige Geräte benötigt.

Optische Datenspeicher, die unter Verwendung von organischen Verbindungen arbeiten sind ebenfalls bereits bekannt und in EP-A 159 397, US 3,668,663, US 3,683,336, US 3,720,921, DE-A 24 46 700 oder auch DE-A 22 10 050 beschrieben.

Es wurde nun gefunden, daß die Herstellung von leicht beschreibbaren und vorzugsweise auch löschbaren optischen Datenspeichern mit hervorragender Langzeitstabilität möglich ist, wenn als Speichermedium ein Farbstoff mit mindestens zwei, vorzugsweise zwei, verschieden gefärbten Modifikationen verwendet wird, von denen eine Modifikation durch Zufuhr von Energie, wie z.B. thermischer Energie, Lichtenergie oder Ultraschallenergie, in eine anders gefärbte Modifikation umgewandelt werden kann. Bei zwei verschieden gefärbten Modifikationen kann die eine Modifikation farblos, und die andere Modifikation kann gefärbt bzw. farbig sein, oder es können beide Modifikationen verschieden farbig sein. Entsprechende Voraussetzungen finden sich z.B. bei Farbstoffen mit mindestens zwei, vorzugsweise zwei, verschieden gefärbten allotropen Modifikationen. Jeder der beiden verschieden gefärbten Modifikationen kann dann ein verschiedener Zustand der Informationsspeicherung (logisch 0 oder 1) zugeordnet werden. Beim Einlesen der Informationen wird durch Zufuhr von Energie, vorzugsweise thermischer und/oder Lichtenergie, die eine Modifikation in die andere Modifikation und dadurch gleichzeitig ein logischer Zustand in den anderen, umgewandelt. Nach dieser Umwandlung kann die gespeicherte Information optisch ausgelesen werden. Normalerweise wird dem Zustand der Informationsspeicherung logisch 0 die thermodynamisch stabile oder stabilere Modifikation der Substanz oder des Farbstoffs und dem Zustand der Informationsspeicherung logisch 1 die thermodynamisch metastabile Modifikation der Substanz bzw. des Farbstoffs zugeordnet. Bei den verwendeten Farbstoffen werden dabei die Informationen im Kristallgitter des Farbstoffs gespeichert. Damit die gespeicherten Informationen über lange Zeit stabil bestehen bleiben, werden solche Farbstoffe bevorzugt, bei denen die Umwandlung der metastabilen Modifikation in die stabile Modifikation die Überwindung einer genügend großen Energie-Barriere voraussetzt.

Bei der Verwirklichung von optischen Datenspeichern mit derartigen Farbstoffen, die in zwei verschieden gefärbten, insbesondere festen und vorzugsweise kristallinen, Modifikationen vorliegen, muß beim Lesevorgang in allen Teilen des Sichtweges eine optische Qualität vorliegen. Auch müssen Vorkehrungen getroffen werden, daß durch Streulicht keine Probleme auftreten. Es ist deshalb zweckmäßig, Farbstoffe mit mindestens einer fluoreszierenden Modifikation zu verwenden. Dabei kommen für das Einlesen der Informationen folgende Fälle in Betracht:
1. Umwandlung einer nicht fluoreszierenden Modifikation in eine fluoreszierende Modifikation,
2. Umwandlung einer fluoreszierenden Modifikation in eine nicht fluoreszierende Modifikation und
3. Umwandlung einer fluoreszierenden Modifikation in eine andere, in einem anderen Spektralbereich fluoreszierende Modifikation.

Für das optische Auslesen der gespeicherten Informationen sind die vorstehenden Fälle 1 und 2 gleichwertig. Bei dem vorstehend genannten 3. Fall ist bei der Auslesung die Ausfilterung eines der beiden Spektralbereiche des Fluoreszenzlichts erforderlich.

Den erfindungsgemäßen Verbindungen ähnliche Verbindungen sind bereits bekannt und in EP-A 133 156, EP-A 181 290, US 4,415,685, Chem.Ber. 120, 1075(1987) oder Angew. Chem. 99, 566(1987) beschrieben.

Geeignete erfindungsgemäße Substanzen sind Farbstoffe der allgemeinen Formel I 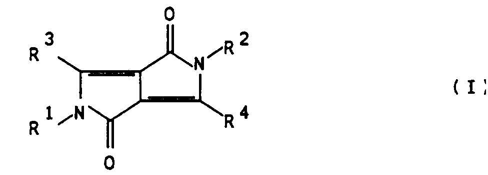 worin R¹ und R unabhängig voneinander (C₁-C₄)-Alkyl und R³ und R⁴ ortho-(C₁-C₄)-Alkoxyphenyl bedeuten.

In der allgemeinen Formel I bedeuten R³ und R⁴ bevorzugt ortho-Methoxyphenyl.

Besonders bevorzugt bedeuten in der allgemeinen Formel I R¹ und R Methyl, R³ ortho-Methoxyphenyl und R⁴ ortho-Methoxyphenyl.

Ganz besonders bevorzugt ist der Farbstoff der Formel III

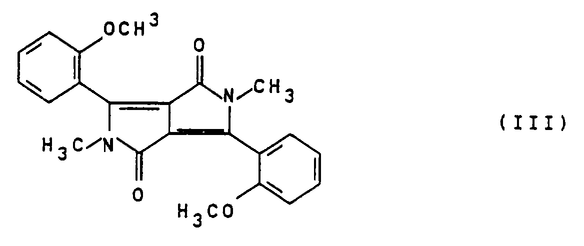

Das 3,6-Bis-(2'-methoxyphenyl)-2,5-dihydro-2,5-dimethylpyrrolo-(3,4-c)-pyrrol-1,4-dion der Formel III kristalliert aus organischen Lösungsmitteln in einer orangefarbenen Modifikation, die nur verschwindend wenig fluoresziert. Diese Modifikation wird im folgenden IIIb genannt. Erhitzt man diese Modifikation auf 195 bis 200°C, dann wandelt sie sich rasch in eine thermodynamisch stabilere gelbe Modifikation IIIa um, die eine intensive gelbgrüne Feststofffluoreszenz besitzt. Diese thermische Umwandlung eignet sich hervorragend für Speicherzwecke. Von besonderem Vorteil ist dabei die hohe Beständigkeit der Farbstoffe IIIa und IIIb. Auch die photochemische Beständigkeit der Farbstoffe ist ausgesprochen hoch, denn sie haben intensive Sonnenbestrahlung ohne irgendein Anzeichen einer Veränderung bis jetzt mehrere Monate lang überstanden. Die Farbstoffe IIIa und IIIb sind daher nach bisherigen Erfahrungen unbegrenzt haltbar. Insbesondere wird keinerlei lichtinduzierte Umwandlung der orangefarbenen Modifikation IIIb in die gelbe Modifikation IIIa beobachtet. Dies ist für eine große Zahl von Lesezyklen eines optischen Speichers wichtig. Die Umwandlungsenthalpie von IIIb in IIIa wurde bestimmt und beträgt -1,5 kcal/mol. Da die Reaktion exotherm ist, wird sie nach Überschreiten einer kritischen Temperatur freiwillig weiterlaufen. Dies vereinfacht den Schreibvorgang und macht ihn störsicherer, weil dadurch leicht eine vollständige Umwandlung des Farbstoffs in die andere Modifikation und damit in den anderen logischen Zustand zu erreichen ist. Die dabei freiwerdende Enthalpie ist aber mit -1,5 kcal/mol so klein, daß keinerlei Beeinträchtigung einer verwendeten Matrix durch die Wärmeentwicklung erfolgt und die Umwandlung einer Speicherstelle auch benachbarte Speicherstellen nicht beeinflußt. Die Kristallgitter beider Modifikationen sind sehr beständig. Die Farben der Substanzen und die Reflexe von Röntgen-Pulveraufnahmen verändern sich auch bei intensiver mechanischer Beanspruchung nicht. Die Grundbedingungen für einen optischen Fluoreszenz-Datenspeicher werden damit vom Farbstoff III in nahezu idealer Weise erfüllt.

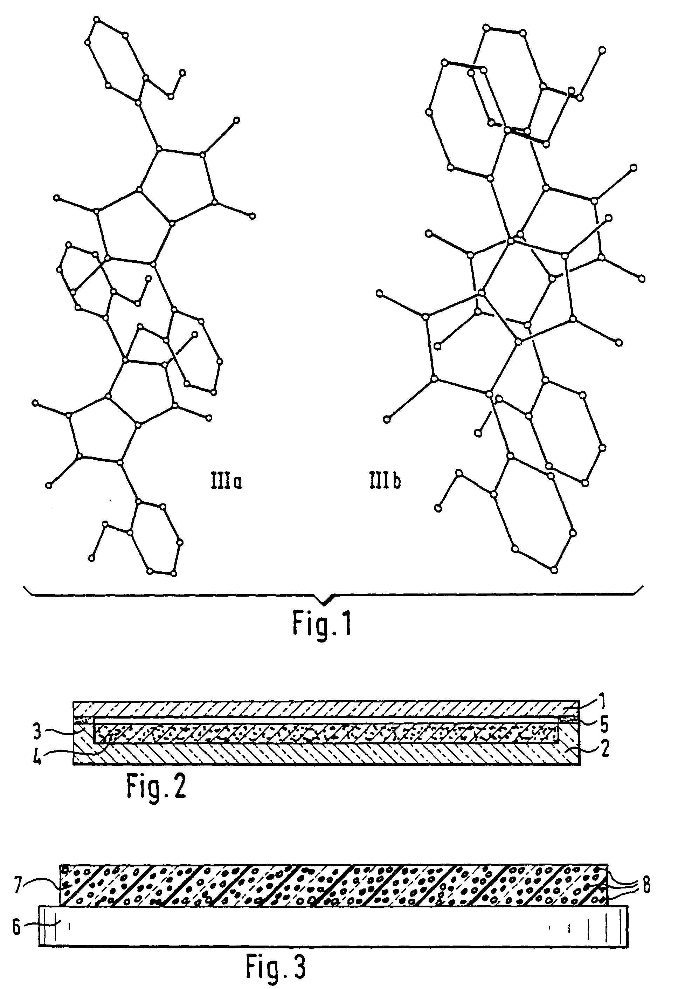

Nach Röntgenkristalluntersuchungen kristallisieren die Modifikationen IIIa und IIIb monoklin. Ihre kristallographischen Dichten unterscheiden sich nur verschwindend wenig. Bei IIIa ist die Methoxygruppe zur Carbonylgruppe des Chromophors gedreht, so daß der o-Anisylrest in einem Winkel von 59° zur Ebene des Chromophors steht. Bei IIIb ist die Methoxygruppe der Carbonylgruppe abgewandt, der entsprechende Winkel beträgt hier 50°. In beiden Kristallgittern sind die Chromophore ähnlich gestapelt, insbesondere werden keine Dimerenpaare gefunden. Informationen über die für die Fluoreszenz wesentlichen Wechselwirkungen der Chromophore werden aus Röntgenstrukturanalysen der beiden Modifikationen erhalten. Ein Ausschnitt der Kristallgitter mit einer Blickrichtung senkrecht auf die Ebene der Chromophore ist in Figur 1 angegeben. Für die nur schwach fluoreszierende, orangefarbene Modifikation IIIb sieht man dort die Chromophore direkt übereinander gelagert. Bei einem Ebenenabstand von nur 3,81 Å tritt eine intensive Wechselwirkung der Chromophore auf, die zum einen zu einer bathochromen Verschiebung der Absorption führt (orangefarben) und zum anderen eine starke Ankopplung der Elektronenanregung an Gitterschwingungen bewirkt. Durch die letztere fließt die Anregungsenergie in Gitterschwingungen ab, und eine Feststofffluoreszenz wird weitgehend gequenscht. Bei der Modifikation IIIa dagegen sind die Chromophore stark gegeneinander verschoben, so daß eine Chromophor-Chromophor-Wechselwirkung erst wieder mit der übernächsten Schicht erfolgen kann. Diese ist aber bereits 6.18 Å entfernt - die Wechselwirkungen werden daher vernachlässigbar klein. Die im wesentlichen isolierten Chromophore verhalten sich im Kristall nun wie in verdünnter Lösung. Sie absorbieren langwellig (gelb) und fluoreszieren stark.

Gegen einen intramolekularen Elektronentransfer von der Methoxygruppe zum Chromophor spricht zum einen, daß bei IIIb deren Abstand größer zur Carbonylgruppe ist als bei IIIa und zum anderen in Lösung keine nennenswerte Abhängigkeit der Fluoreszenzquantenausbeute von der Lösungsmittelpolarität beobachtet wird. Die Fluoreszenzquantenausbeuten in Chloroform und Acetonitril unterscheiden sich nur wenig. Der geringe Abstand und damit die stärkeren Wechselwirkungen der Chromophore in IIIb spiegeln sich auch in den Feststoff-Absorptionsspektren wieder, denn das orangefarbene IIIb absorbiert wesentlich längerwellig als das gelbe IIIa.

Es ist anzunehmen, daß der geringe Abstand der Chromophore in IIIb nicht nur zu einer langwelligen Absorption führt, sondern auch zu einer festen Ankoppelung des Elektronensystems an Gitterschwingungen, denn eine periodische Änderung des Chromophorabstands bedingt eine periodische Änderung des UV-VIS-Absorptions- bzw. Fluoreszenzspektrums. Über die Kopplung kann die elektronische Anregungsenergie in Gitterschwingungen abfließen. Der Prozeß der Internal Conversion wird damit gefördert. Diese Fluoreszenzdesaktivierung hat dagegen beim Farbstoff IIIa durch den größeren Abstand der Chromophore keine Bedeutung. Die Wechselwirkung ist bereits klein und wird durch Gitterschwingungen kaum beeinflußt. Dies erklärt die starke Feststofffluoreszenz des Farbstoffs.

Bei einem erfindungsgemäßen optischen Fluoreszenz-Datenspeicher ist der erfindungsgemäß zu verwendende Farbstoff mit mindestens einer fluoreszierenden Modifikation auf dem Datenträger, z.B. einer Platte, aufgebracht und fixiert oder in dem Datenträger suspendiert. Insbesondere ist der Datenspeicher mit dem bevorzugt verwendeten Farbstoff der Formel III beschichtet, oder er enthält diesen Farbstoff. Die zu speichernden Informationen werden dann z.B. mit einem Laserstrahl eingeschrieben, wodurch eine thermische Umwandlung des Farbstoffs IIIb in den Farbstoff IIIa erfolgt. Zum Auslesen der Informationen wird die Platte z.B. mit einem Laserstrahl abgetastet, der den Farbstoff IIIa an den umgewandelten Stellen zur Fluoreszenz anregt. Die Detektion kam einfach durch eine Abbildung des fluoreszierenden Punkts auf einen lichtempfindlichen Empfänger erfolgen. Die Abklingzeit der Fluoreszenz ist mit ca 10⁻⁸ bis 10⁻⁹s so kurz, daß mit hoher Frequenz ausgelesen werden kann.

Ein derartiger Fluoreszenzspeicher kann einmal beschrieben und dann sehr häufig ausgelesen werden. In der vorliegenden Form besteht aber keine Möglichkeit, ihn zu löschen. Löschbare Speicher werden aber insbesondere in der Computertechnik bevorzugt.

Löschbare optische Datenspeicher, insbesondere Fluoreszenz-Datenspeicher, lassen sich ebenfalls unter Verwendung erfindungsgemäß zu verwendender Farbstoffe, insbesondere unter Verwendung des Farbstoffs der Formel III, herstellen.

Um einen z.B. mit dem Farbstoff III als Datenspeicher realisierten, beschriebenen optischen Fluoreszenz-Datenspeicher zu löschen, muß der Farbstoff IIIa wieder in die Form IIIb umgewandelt werden. Um dies zu erreichen, kann die große Kristallisationstendenz der Modifikation IIIb ausgenutzt werden, denn aus homogener Lösung kristallisiert der Farbstoff III freiwillig in der thermodynamisch ungünstigen Modifikation IIIb aus. Mischt man nun den Farbstoff mit einer thermisch stabilen Hilfssubstanz, die hinreichend hoch, z.B. wenig oberhalb des Umwandlungspunkts, von ca. 170 bis 190°C, schmilzt, dann kann diese Schmelze den Farbstoff auflösen. Beim Abkühlen kristallisiert dann IIIb aus, und der thermisch eingeschriebene Speicherpunkt ist wieder gelöscht. Die hochschmelzende Hilfssubstanz sollte thermisch stabil sein.Als derartige Hilfssubstanz sind z.B. hochschmelzende Aromaten geeignet.

Besonders gut geeignet als hochschmelzende Hilfssubstanz sind Anthracen, insbesondere Reinstanthracen, und insbesondere Terphenyl. Anthracen z.B. bildet mit dem Farbstoff III keine Mischkristalle. Mischt man z.B. den Farbstoff III in der Modifikation IIIb im Gewichtsverhältnis 1 : 5 mit Anthracen, so erhält man ein orangefarbenes Pulver, in dem der Farbstoff nicht oder nur sehr schwach fluoresziert. Erhitzt man dieses Gemisch nun auf Temperaturen von 170 bis 195°C, dann wandelt sich im Feststoff die Modifikation IIIb in IIIa um, und die Substanz fluoresziert stark gelbgrün. Die Umwandlung, dies entspricht nun dem Einschreiben einer Information, kann nun über die Fluoreszenz ausgelesen werden. Erhitzt man dann das Gemisch weiter auf über 220°C, d.h. bis über den Schmelzpunkt des Anthracens hinaus, dann schmilzt das Anthracen und löst den Farbstoff III, gleichgültig ob er in der Form IIIa oder IIIb vorliegt, auf. Bei Abkühlen kristallisiert dann die nicht fluoreszierende Modifikation IIIb aus. Die Information ist wieder gelöscht. Dieser Zyklus kann ohne Veränderung des Farbstoffs oder des Anthracens als Hilfsstoff oder Matrix viele Male durchlaufen werden und ist nur durch die thermische Langzeitstabilität der Komponenten begrenzt.

Anthracen eignet sich als Material für die Hilfssubstanz bzw. die Matrix sehr gut, da es zum einen leicht durch eine chromatographische Filtration mit Chloroform an Aluminiumoxid hochrein hergestellt werden kann und zum anderen als aromatischer Kohlenwasserstoff genügend thermisch stabil und inert ist. Darüberhinaus liegt sein Schmelzpunkt im optimalen Temperaturbereich. Als Hilfsstoff besonders gut geeignet ist auch Terphenyl. Andere aromatische Kohlenwasserstoffe können nach eventuell geeigneter Substitution ebenfalls als Hilfssubstanz bzw. Matrixmaterial eingesetzt werden. Auch Heterocyclen sind hierfür geeignet. Weitere Beispiele für geeignete hochtemperaturstabile aromatische Kohlenwasserstoffe sind: Biphenyl, Quaterphenyl und lineare und verzweigte Polyphenylene mit einem Polymerisationsgrad von mindestens 3 bis 500, bevorzugt 3 bis 50, stärker bevorzugt 3 bis 10. Weiterhin bevorzugt sind die Kohlenwasserstoffe Naphthalin, Phenanthren, Triphenylen, Chrysen, 3,4-Benzophenanthren, 1,2-Benzochrysen, Picen, 5,6-Benzochrysen, 3.4,5.6-Dibenzophenanthren, Hexahelicen, 1.2,7.8-Dibenzochrysen, 11.12,13.14-Dibenzopicen, 1.2,3.4,5.6,7.8,9.10,-11.12-Hexabenzotriphenylen, Tetraphen, 1.2,3.4-Dibenzanthracen, 1.2,5.6-Dibenzanthracen, 1.2,7.8-Dibenzanthracen, 1.2,3.4,5.6-Tribenzanthracen, 1.2,3.4,5.6,7.8-Tetrabenzanthracen, 1.2-Benzotetraphen, 3.4-Benzotetraphen, 3.4,8.9-Dibenzotetraphen, Pentaphen, 3.4-Benzopentaphen, 3.4,9.10-Dibenzopentaphen, 1.2,5.6-Dibenzotetraphen, 6.7-Benzopentaphen, Dinaphtho-(2'.1':1.2);(2".1":5.6)-anthracen, Naphtho-(2'.3':6.7)-pentaphen, Naphtho--(2'.3':3.4)-pentaphen, Anthraceno-(2'.1':1.2)-anthracen, Anthraceno-(2'.1':8.9)-tetraphen, Tetrapheno-(8'.9':8.9)-tetraphen, 2.3-Benzopicen, 2.3,8.9-Dibenzopicen, Anthraceno-(1'.2':1.2)-tetraphen, Fluoren, Biphenylen, 1.2-Benzobiphenylen, 2.3-Benzobiphenylen, 1.2,7.8-Dibenzobiphenylen, 2.3,6.7-Dibenzobiphenylen, Tetraphenylen, Pentaphenlyen, Hexaphenylen, Octaphenylen, Perylen, 1.12-Benzoperylen, Coronen, Pyren, 1.2-Benzopyren, 3.4-Benzopyren, Fluoranthen, 2.3-Benzofluoranthen, 2.3-Benzofluoranthen, 10.11-Benzofluoranthen, 11.12-Benzofluoranthen, 9.10,11.12-Dibenzofluoranthen, 2.13-Benzofluoranthen, 4.5-o-Phenylenfluoranthen, Isorubicen, 10.11-(peri-Naphthylen)-fluoranthen, 11.12-(peri-Naphthylen)-fluoranthen, Decacyclen. Diese Aromaten und das Anthracen und das Terphenyl können ihrerseits wieder 1 bis 4 der unter a) bis r) genannten Reste tragen. Geeignet sind auch substituierte, entsprechend hochschmelzende Derivate des Benzols. Weiterhin sind als Hilfssubstanzen teilkristalline oder flüssigkristalline Polymere, wie z.B. Silikonpolymere, oder Perfluorether mit aromatischen Gruppen geeignet.

Die hochtemperaturstabilen Hilfssubstanzen können einzeln oder als Mischungen mit einem geeigneten Schmelzpunkt verwendet werden.

Eine weitere Möglichkeit für die Realisierung eines löschbaren optischen Fluoreszenz-Datenspeichers besteht darin, daß der in einer Modifikation fluoreszierende Farbstoff, insbesondere der Farbstoff der Formel III, nicht in einer hochschmelzenden Substanz, sondern in einer schlecht lösenden hochsiedenden Flüssigkeit als Medium eingebettet wird, die den Farbstoff bei Zimmertemperatur und auch bei der Temperatur der Umwandlung nur wenig löst, diesen aber dann bei höheren Temperaturen, bei Farbstoff III etwa 300°C, löst. Beim Abkühlen kristallisiert dann ebenfalls wieder die thermodynamisch ungünstigere Modifikation IIIb aus, wodurch, wie beim Anthracen, die Information gelöscht wird. Problematisch ist dabei jedoch die starke Fluoreszenz des Farbstoffs in Lösung, so daß nur sehr wenige schlecht lösende Flüssigkeiten in Frage kommen. Hier hat sich besonders oligomerer Perfluorether als günstig herausgestellt, der auch mit oligomerem Polytrifluorchlorethylen gemischt werden kann. Geeignet sind ferner: Polytetrafluorethylene, insbesondere solche mit einem Polymerationsgrad von 10 bis 100.000, Perfluorpolyetheröle, insbesondere solche mit einem Polymerisationsgrad von 10 bis 100.000, ein Gemisch aus Perfluorpolyetheröl und Polyvinylidenfluorid mit Polymerisationsgraden von 10 bis 100.000 sowie Silikonöle und Silikonharze.

Die erfindungsgemäßen optischen Datenspeicher enthalten als Speichermedium einen Farbstoff mit mindestens zwei, vorzugsweise zwei, verschieden gefärbten Modifikationen, von denen eine Modifikation durch Zufuhr von Energie, z.B. in Form von Ultraschall, Wärme oder vorzugsweise Licht, in eine anders gefärbte Modifikation umgewandelt werden kann. Bevorzugt sind Fluoreszenzdatenspeicher, die eine in mindestens einer Modifikation fluoreszierende Substanz, vorzugsweise den Farbstoff III, als Speichermedium enthalten. Ganz besonders bevorzugt sind Datenspeicher, die nicht nur einmal beschrieben und dann beliebig oft gelesen, sondern die auch wieder gelöscht werden können. Derartige besonders bevorzugte erfindungsgemäße Datenspeicher enthalten erfindungsgemäß zu verwendende Substanzen als Speichermedium, die nicht nur durch Energiezufuhr von einer Modifikation in eine anders gefärbte umgewandelt werden können, sondern die auch wieder von der zweiten in die erste Modifikation umgewandelt werden können. Derartige löschbare, insbesondere Fluoreszenzdatenspeicher enthalten das Speichermedium evtl. zusammen mit einer Hilfssubstanz oder einem Hilfssubstanzgemisch der bereits genannten Art.

Als Träger für die erfindungsgemäß zu verwendenden Substanzen können z.B. anorganische Gläser, wie z.B. Silikat-, Phosphat- oder Boratgläser, amorphes Siliciumdioxid, organische Gläser, wie z.B. Polymethacrylat oder Polycarbonat oder auch andere, gegebenenfalls auch teilkristalline Materialien wie Polyvinylchlorid, Polystyrol, Polyethylen, Polypropylen, Polyvinylidenchlorid, Polyester und Polyamide verwendet werden.

Die erfindungsgemäß zu verwendenden Farbstoffe können z.B. in einer geeigneten Weise auf einen Träger aufgebracht und fixiert werden. Das Aufbringen erfolgt beispielsweise mit Siebdruckverfahren oder mit Aufdampftechniken, zweckmäßigerweise in einem bestimmten Muster, so daß getrennte Speicherstellen vorliegen, die leicht getrennt adressierbar und später wieder auslesbar sind. Für das Aufbringen der Substanzen, insbesondere Farbstoffe, in einem bestimmten Muster auf einen Träger und ihrer dann auf dem Träger erfolgenden Fixierung sind jedoch auch noch andere Techniken geeignet, insbesondere eine Kristallisation aus einer Lösung der Substanz in einem organischen Lösungsmittel. Dabei wird insbesondere ein solches organisches Lösungsmittel als Medium verwendet, das einen Schmelzpunkt oberhalb des Umwandlungspunktes der benutzten Substanz hat und diese dann im geschmolzenen Zustand löst. Insbesondere wird ein solches organisches Lösungsmittel als Medium verwendet, das im geschmolzenen Zustand die Substanz löst, aber sie beim Abkühlen in einer metastabilen Modifikation auskristallisieren läßt. Ein derartiges Medium sind beispielsweise Anthracen und Terphenyl.

Die erfindungsgemäß als Speichermedien zu verwendenden Substanzen können auch in dem Träger suspendiert oder in anderer Weise in dem Träger enthalten sein.

Es ist zweckmäßig, die als Datenspeicher zu verwendenden Substanzen in einer sehr einheitlichen Korn- bzw. Kristallgröße, beispielsweise im Bereich von 0,1 bis 200µm, vorzugsweise von 0,1 bis 50µm, und ganz besonders bevorzugt von 1 bis 10µm, in die genannten Trägermaterialien einzubringen oder auf die Trägermaterialien aufzubringen.

Die aus dem Träger und dem erfindungsgemäß zu verwendenden Speichermedium und gegebenenfalls Hilfsstoff oder Hilfsstoffgemisch bestehenden erfindungsgemäßen Datenspeicher können z.B. die Form einer drehbaren Scheibe besitzen, wie sie in Disk-Systemen verwendet wird, oder sie können z.B. auch eine stationäre Platte bilden. Bevorzugt sind Fluoreszenz-Datenspeicher, die einen in mindestens einer Modifikation, und vorzugsweise einen nur in einer Modifikation fluoreszierenden Farbstoff, insbesondere den Farbstoff III enthalten. Erfindungsgemäße Datenspeicher enthalten die als Speichermedien zu verwendenden Substanzen auf einem geeigneten Träger oder in einem geeigneten Träger, evtl. zusammen mit einer Hilfssubstanz, wie Anthracen oder Terphenyl, oder Hilfssubstanzgemisch. In die in oder auf dem Träger vorliegenden als Speichermedium verwendeten Substanzen lassen sich Informationen durch Zufuhr von Energie, insbesondere thermischer Energie oder Lichtenergie, einschreiben. Die Zufuhr thermischer Energie kann z.B. durch Thermoprintersysteme erfolgen. Das Einschreiben der Informationen erfolgt jedoch vorzugsweise mit einem Laserstrahl. Das nach dem Einschreiben der Informationen erfolgende Auslesen kann mit Hilfe eines Lichtstrahls erfolgen, wobei die Absorption der verwendeten Farbstoffe als Kriterium für eine Speicherung verwendet wird. Bei der Auslesung der Informationen mit einem Lichtstrahl wird jedoch vorzugsweise die Fluoreszenz der verwendeten Farbstoffe als Speicherkriterium verwendet. Dabei kann das auftretende Fluoreszenzlicht nach bekannten Verfahren auf ein lichtempfindliches elektronisches Bauelement fokusiert werden. Das auftretende Fluoreszenzlicht kann auch so gefiltert werden, daß von dem anregenden Lichtstrahl kein Streulicht auf den Empfänger des Fluoreszenzlichts fällt. Derartige nicht löschbare und löschbare optische Speicher können z.B. in EDV-Systemen, insbesondere Massespeichern von Computern, Textverarbeitungsgeräten, Bildverarbeitungsgeräten und Bildaufzeichnungssystemen oder in Audio-Speichersystemen, wie z.B. vorzugsweise digitalen Plattenspielern, oder in Videosystemen, vorzugsweise in Videorekordern und Bildplatten, oder in holographischen Systemen verwendet werden.

Zum Auslesen löschbarer optischer Fluoreszenz-Datenspeicher wird ein Laser benötigt, der in der Absorption des Farbstoffs einstrahlt, also z.B. eine Wellenlänge von ca. 515 nm oder weniger besitzt. Laser mit diesem Wellenlängenbereich stehen zur Verfügung. Um das System weiter zu vereinfachen, kann man Halbleiterlaser verwenden, die mit ihrer intensiven IR-Strahlung beim Schreibvorgang die umgehende Matrix aufheizen. Beim Lesevorgang kann man dann in bekannter Weise die Frequenz des Lasers durch das Einschalten eines Kristalls in den Lichtweg verdoppeln, so daß man nun mit niedrigerer Intensität Licht mit der halben Wellenlänge zum Anregen der Fluoreszenz erhält. Bei einer Verwendung der kürzeren Wellenlänge des Lichts erhält man ein größeres Auflösungsvermögen bei der Datenspeicherung.

Eine weitere Möglichkeit für den Bau von Fluoreszenz-Datenspeichern bietet der Einsatz von solchen Farbstoffen der Formel I, die Lösungsmittelmoleküle in das Kristallgitter einbauen. Bei hohen Temperaturen wird dieses Lösungsmittel dann wieder in Freiheit gesetzt, und die Farbe und eventuell auch die Fluoreszenz wird geändert.

Farbstoffe, die durch den Einbau von Lösungsmitteln in das Kristallgitter eine andere Farbe und/oder andere Fluoreszenz erhalten, können in optischen Datenspeichern verwendet werden, die einmal beschrieben werden. Das Lösungsmittel wird bei dem Vorgang des Beschreibens abgegeben.

Erfindungsgemäße bevorzugte beschreib- und löschbare Fluoreszenz-Datenspeicher sind in den Figuren 2 und 3 beispielsweise und schematisch dargestellt.

Die Figur 2 zeigt einen Vertikalschnitt durch einen Datenspeicher in Form einer runden Platte oder Scheibe. Diese setzt sich zusammen aus der oberen Scheibe 1 und der unteren Scheibe 2, die einen umlaufenden hochgezogenen Rand 3 besitzt. Dadurch wird eine Schale gebildet, in die z.B. ein Gemisch des Farbstoffs III und eine der genannten Hilfssubstanzen, wie z.B. Reinstanthracen z.B. im Gewichtsverhältnis 1 : 5 eingefüllt wird. In der Figur 1 ist dieses Gemisch mit 4 bezeichnet. Die obere Scheibe 1 wird dann auf den Rand 3 der unteren Scheibe 2 aufgelegt und fest mit ihm verbunden, z.B. verklebt (5). Mindestens eine der Scheiben 1 und 2 besteht aus einem optisch durchlässigen Material. Die Scheiben 1 und 2 können z.B. aus Glas bestehen. Durch Erhitzen auf z.B. 220°C wird das Gemisch 4 geschmolzen, und aus der Schmelze kristallisiert beim Abkühlen die thermodynamisch metastabile Modifikation III b des Farbstoffs III aus. Der Datenspeicher kann nun in der bereits geschilderten Weise beschrieben, ausgelesen und gewünschtenfalls auch wieder gelöscht werden.

Die Figur 3 zeigt einen Vertikalschnitt durch einen anderen Datenspeicher in Form einer Platte oder Scheibe. Diese besteht aus dem Träger 6, auf den eine Schicht 7 aufgebracht ist. Diese Schicht 7 besteht aus einem optisch durchlässigen Kunststoff, z.B. aus Polyamid oder Polyimid, in die gleichmäßig verteilte feine Teilchen, insbesondere Kügelchen 8 eingelagert sind. Diese Teilchen oder Kügelchen 8 entstehen z.B. aus einem Gemisch aus Anthracen oder Terphenyl und einem geeigneten Farbstoff, wie z.B. dem Farbstoff III. Die Teilchen oder Kügelchen 8 werden in dem Kunststoff gleichmäßig verteilt, und der Kunststoff mit den Kügelchen auf den Träger 6 aufgebracht, was z.B. im Schleuderverfahren erfolgen kann. Gewünschtenfalls kann die Schicht noch durch eine (in der Figur 3 nicht dargestellte) Abdeckschicht aus optisch durchlässigem Material, z.B. einem geeigneten Kunststoff, abgedeckt sein. Der in Figur 3 schematisch dargestellte Datenspeicher kann auf die bereits genannte Art und Weise beschrieben, ausgelesen und gelöscht werden.

Bei einem erfindungsgemäßen optischen Fluoreszenz-Datenspeicher erfolgt bei einer Fokussierung des auslesenden Lichtstrahls auf das aus einem Fluoreszenzfarbstoff gebildete Speicherelement die Fluoreszenz nahezu isotrop in alle Raumrichtungen; das auszulesende Speicherelement wird selbst zur Lichtquelle. Da die Adressierung der Speicherelemente räumlich und zeitlich durch den Anregungsstrahl bestimmt wird, reicht eine verhältnismäßig einfache Optik für das Detektionssystem aus. Auch optische Inhomogenitäten des Speicherelements wirken sich nur sehr wenig auf dessen Lesbarkeit aus. Darüberhinaus erfolgt wegen des Stokes-Shifts der Fluoreszenzfarbstoffe die Lichtabgabe bei einer längeren Wellenlänge als die Anregung. Unkontrollierte Reflexionen des Anregungslichts können dadurch auf einfach Weise ausgefiltert werden. Die Störanfälligkeit des Systems wird dadurch noch weiter verringert. Bei den erfindungsgemäßen optischen Datenspeichern ist die erzielbare Speicherdichte praktisch nur durch die zum Ein- bzw. Auslesen benutzte Lichtwellenlänge begrenzt. Die erzielbare Speicherdichte ist daher hoch. Die erfindungsgemäßen optischen Datenspeicher besitzen eine große Langzeitstabilität und ermöglichen dadurch und weil kein partielles Löschen durch den Lesevorgang stattfindet eine praktisch unbegrenzte Zahl von Lesezyclen. Die Lesegeschwindigkeit ist hoch (100 MHz bis 16 GHz, entsprechend 1 bis 10ns). Die Schreib- und Löschgeschwindigkeit ist mittel bis hoch.

Die Verbindungen der allgemeinen Formel I können nach verschiedenen, an sich bekannten Verfahren hergestellt werden. Nach dem in der EP-B1-61 426 beschriebenen Verfahren wird ein Nitril der Formel R³CN, gegebenenfalls zusammen mit einem Nitril der Formel R⁴CN, Bromessigester und Zink umgesetzt, wobei eine Verbindung der Formel I mit R¹ = R = H entsteht. Bessere Ausbeuten werden erhalten, wenn in an sich bekannter Weise 2 Mol des Nitrils R³CN oder des Nitrilgemischs R³CN/R⁴CN mit 1 Mol eines Bernsteinsäurediethylesters in einem organischen Lösungsmittel in Gegenwart einer starken Base bei erhöhter Temperatur umgesetzt werden, vgl. EP-B1-94 911 und T. Potrawa u.H. Langhals, Chem. Ber. 1920, 1075-1078 (1987).

Ein besonders günstiges Verfahren zur Herstellung der Verbindungen der allgemeinen Formel I besteht darin, daß Furanofurandion der allgemeinen Formel V 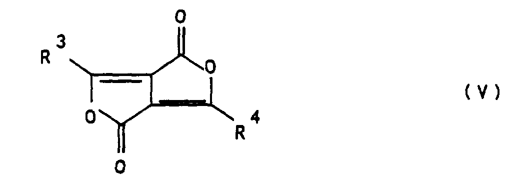 mit einem primären Amin der allgemeinen Formel R¹NH₂ oder mit einem Gemisch primärer Amine R¹NH₂/RNH₂ umgesetzt wird. Dabei besitzen R¹, R, R³ und R⁴ die bereits genannten Bedeutungen. (Falls R¹ und/oder R Wasserstoff bedeuten, handelt es sich bei dem primären Amin R¹NH₂ bzw. RNH₂ um Ammoniak. Es werden dann Verbindungen der Formel I mit R¹ und/oder R = H erhalten. Das Ammoniak kann auch in Form von Ammoniak abspaltenden Verbindungen eingesetzt werden).

Die Umsetzung wird zwischen der Verbindung der allgemeinen Formel V und dem primären Amin oder dem Gemisch primärer Amine zweckmäßigerweise in einem geeigneten inerten Lösungsmittel oder Dispergiermittel durchgeführt.

Geeignete Lösungs- oder Dispergiermittel sind z.B. Ether, insbesondere solche mit 2 bis 8 C-Atomen im Molekül, wie z.B. Diethylether, Methylethylether, Di-n-propylether, Diisopropylether, Methyl-n-butylether, Methyl-tert-butylether, Ethyl-n-propylether, Di-n-butylether, Tetrahydrofuran, 1,4-Dioxan, 1,2-Dimethoxyethan, Bis-β-methoxyethylether; Oligoethylen-glykol-dimethylether, wie z.B. Pentaglyme; aliphatische Kohlenwasserstoffe, wie z.B. Hexan, Heptan, niedrig- und hochsiedende Petrolether; cycloaliphatische Kohlenwasserstoffe, wie z.B. Cyclohexan, Methylcyclohexan, Tetralin, Decalin; aromatische Kohlenwasserstoffe, wie z.B. Benzol, Toluol, o-, m- und p-Xylol, Ethylbenzol; halogenierte aliphatische oder aromatische Kohlenwasserstoffe, wie z.B. Methylenchlorid, Chloroform, Tetrachlorkohlenstoff, Chlorbenzol, Dichlorbenzol; Nitrile, wie z.B. Acetonitril; Amide, wie z.B. Dimethylformamid, Dimethylacetamid, N-Methyl-pyrrolidon; Hexamethylphosphorsäuretriamid; Sulfoxide, wie z.B. Dimethylsulfoxid. Auch Gemische verschiedener Lösungsmittel können verwendet werden.

Die Durchführung der Reaktion in einem dipolar aprotischen Lösungsmittel ist bevorzugt. Bevorzugte dipolar aprotische Lösungsmittel sind z.B.: Dimethylformamid, Dimethylsulfoxid, Hexamethylphosphorsäuretriamid, Sulfolan, N-Methylpyrrolidon, Tetramethylharnstoff, Acetonitril, Ethylenglykoldimethylether, Ethylenglykoldiethyl-ether, Diethylenglykoldimethylether und Triethylenglykoldimethylether, Nitromethan, 1,3-Dimethyl-3,4,5,6-tetrahydro-2(1H)-pyrimidinon (DMPU). 1,3-Dimethyl-2-imidazolidinon, Benzonitril, Nitrobenzol, Chloroform, Tetrachlorkohlenstoff und Methylenchlorid. Besonders bevorzugte dipolar aprotische Lösungsmittel sind Chloroform, Tetrachlorkohlenstoff und Methylenchlorid, von denen Chloroform ganz besonders bevorzugt ist.

Es ist weiterhin zweckmäßig, die Reaktion zwischen der Verbindung der allgemeinen Formel V und dem primären Amin oder dem Gemisch primärer Amine in Gegenwart eines wasserentziehenden Mittels durchzuführen. Als derartige wasserentziehende oder wasserabspaltende Mittel sind z.B. geeignet: N,N'-disubstituierte Carbodiimide, insbesondere wenn sie mindestens einen sekundären oder tertiären Alkylrest enthalten, wie z.B. Diisopropyl-, Dicyclohexyloder N-Metyhl-N'-tert.butyl-carbodiimid (vgl. Methodicum Chimicum, Verlag G.Thieme, Stuttgart, Bd.6, (1974), S.682/683, und Houben-Weyl, Methoden der Org. Chemie, Bd.8, (1952), S. 521/522). Dicyclohexylcarbodiimid ist ganz besonders geeignet.

Die Umsetzung zwischen der Verbindung der Formel V und dem primären Amin R¹NH₂ oder dem Gemisch der primären Amine R¹NH₂/RNH₂ kann z.B. bei Temperaturen von -10°C bis zum Siedepunkt des verwendeten Lösungsmittels oder Lösungsmittelgemischs durchgeführt werden. In vielen Fällen wird sie bei 10 bis 30°C und vorzugsweise bei Raumtemperatur durchgeführt. Pro Mol Furanofurandion der allgemeinen Formel V werden zweckmäßigerweise 1,9 bis 2,4 mol, vorzugsweise 2,0 bis 2,3 mol des primären Amins R¹NH₂ oder des Gemisches primärer Amine R¹NH₂/RNH₂ eingesetzt.

Die Ausgangsverbindungen der allgemeinen Formel V und die primären Amine R¹NH₂ und RNH₂ sind bekannt oder können nach den für die Herstellung dieser Verbindungsklassen bekannten Methoden leicht hergestellt werden.

Weitere Verfahren zur Herstellung von Verbindungen der allgemeinen Formel I sind bekannt und z.B. beschrieben in EP-A3-133 156 (Seite 10 ff), EP-A3-181 290, EP-98 808. Bei einem dieser Verfahren, das sich besonders gut für die Herstellung der Verbindungen der Formel I, vorzugsweise der Formeln III und IV, eignet, wird ein Lactam der Formel VI 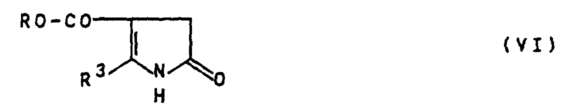 worin R z.B. einen niederen Alkylrest, insbesondere Ethyl, bedeutet und R³ die bereits genannte Bedeutung besitzt, mit einem Nitril der Formel R⁴CN, worin R⁴ die bereits genannte Bedeutung besitzt, umgesetzt, vergl. EP-13-181290, Seite 2. Das Lactam der Formel VI ist z.B. ausgehend von der Dicarbonylverbindung VII über den Acylbernsteinsäureester VIII in an sich bekannter Weise leicht zugänglich. 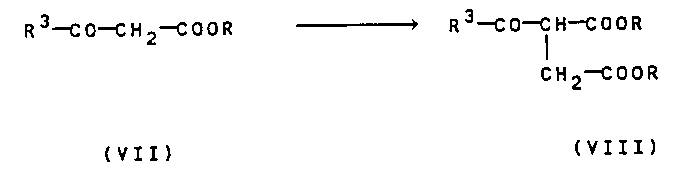 Sofern nach den genannten Verfahren Verbindungen der allgemeinen Formel I mit R¹ = R = H entstehen, können diese Verbindungen in an sich bekannter Weise in Verbindungen der Formel I mit R¹ und/oder R ungleich Wasserstoff durch Umsetzung mit einer die Reste R¹ bzw. R (wobei R¹ und R die bereits genannten Bedeutungen mit Ausnahme von Wasserstoff besitzen) als Abgangsgruppe enthaltenden Verbindungen, zweckmäßigerweise in einem geeigneten inerten Lösungsmittel, umgewandelt werden. Verfahren zur Einführung der Reste R³ und R⁴ (ungleich Wasserstoff) in Verbindungen der Formel I mit R³ = R⁴ = Wasserstoff sind z.B. beschrieben in T. Potrawa u. H. Langhals, Chem. Ber. loc. cit. und EP-13-133158. Geeignete Alkylierungsmittel sind z.B. Alkylhalogenide, insbesondere Alkyliodide, Alkylester, insbesondere Alkylester von Sulfonsäuren, wie z.B. Alkylester der Benzol-oder p-Toluolsulfonsäure.

Die Verbindungen der Formel I lassen sich besonders vorteilhaft durch extraktive Umkristallisation nach dem von H. Langhals in Chem. Ber. 118, (1985), 4641 beschriebenen Verfahren reinigen. Bei diesem Verfahren wird der Extrakt kontinuierlich und zentral in den Inhalt des Extraktionskolbens zurückgeführt, ohne daß dadurch eine Abkühlung des Extraktionskolbeninhalts erfolgt.

Die erfindungsgemäßen optischen Datenspeicher bzw. die Farbstoff der allgemeinen Formel I, insbesondere der Farbstoffe der Formel III, lassen sich allgemein für Markierungszwecke, vorzugsweise für Sicherheitsmarkierungen, einsetzen. Der Vorteil dabei ist, daß in einen gleichmäßig oder ungleichmäßig mit Farbstoff, z.B. mit dem Farbstoff IIIb, beschichteten Träger durch Zufuhr thermischer Energie oder Lichtenergie Informationen eingeschrieben werden können, die dann unter UV-Bestrahlung über die Fluoreszenz erkannt und ausgelesen werden können. Das Einschreiben kann mit einem Laserstrahl erfolgen oder bei noch einfacheren Systemen mit einem Thermoprinter/Plotter. Auch thermisch läßt sich in der aufgebrachten Farbstoffschicht ein Muster sehr schnell und leicht erzeugen. Wichtig ist dies auch für maschinenlesbare Aufdrucke auf Vorlagen, da durch die gezielte Anregung beim Auslesen eine fluoreszierende Markierung, z.B. gegen Verschmutzung, weniger anfällig ist, als eine auf der Absorption beruhende Einfärbung. Ein entsprechend mit Farbstoffen der Formeln I oder II vorbehandeltes Subtrat kann beispielsweise ein Blatt Papier oder eine Kunststoffolie sein, in die thermisch mit einem Laserstrahl oder einem Thermoprinter-Plotter eine fluoreszierende Information eingetragen wird, beispielsweise ein Schriftzug. Dies ist beispielsweise zum Erstelluen einer jeweils spezifischen Sicherheitsmarkierung, für Werbezwecke, zum Markieren von Produkten für spätere Sortiervorgänge und für eine Vielzahl von weiteren Anwendungen von Interesse. Als Beispiele sind noch zu nennen maschinenlesbare Markierungen, vorzugsweise alphanumerische Aufdrucke und Barcodes.

Farbstoffe der Formel I, die als solche oder nach Umkristallisation aus einem organischen Lösungsmittel eine starke Feststofffluoreszenz zeigen, können auch als fluoreszierende Pigmente vielfältig eingesetzt werden, so z.B. als Signalfarben z.B. zum optischen Hervorheben von Schriftzügen und Zeichnungen oder anderen graphischen Produkten, zum Kennzeichnen von Schildern und anderen Gegenständen, bei denen ein besonderer optischer Eindruck erreicht werden soll; als Sicherheitsmarkierung von Scheckkarten oder anderen Gegenständen, bei denen ein besonderer, unverkennbarer Farbeindruck erzielt werden soll; als Zusatz zu anderen Farben, bei denen eine bestimmte Farbnuance durch die Fluoreszenz erzielt werden soll, so z.B. ein besonders leuchtender Farbton; als Markierung von Gegenständen, die maschinell erkannt und sortiert werden sollen und bei denen die Erkennung über die Fluoreszenz erfolgen soll (ein Beispiel hierfür ist das Recycling von Kunststoffen, die mit Hilfe der Fluoreszenz sortiert werden können); als Fluoreszenzfarbstoff für maschinenlesbare Markierungen; als Bestandteil von Tonern für die Elektrophotographie (®Xerox-Verfahren, Laserdrucke), um fluoreszierdende Drucke zu erhalten; für künstlerische Anwendungen und Zwecke; Verwendung in Szintillatoren; Verwendung allgemein für die Frequenzumsetzung des Lichts, z.B.um aus kurzwelligem Licht längerwelliges, sichtbares Licht zu machen; Verwendung als Fluorophor in Beleuchtungs- und Anzeigeelementen, z.B. in Leuchtstoffröhren, in Braunschen Röhren und in Fluoreszenzanzeigen; Verwendung der Farbstoffe in passiven Anzeigeelementen für vielerlei Anzeige-, Hinweis- und Markierungszwecke, z.B. passive Anzeigeelemente, Hinweisund Verkehrszeichen, wie Ampeln; Verwendung in Tintenstrahldruckern.

Farbstoffe der allgemeinen Formel I können auch als thermochrome Temperatursonden verwendet werden. Als Beispiel wird eine Verwendung als Überhitzungskontrolle von Geräten genannt.

### Beispiel 1

### 2,6-Bis(2'-methoxyphenyl)-2,5-dihydro-2,5-dimethylpyrrolo(3,4-c)pyrrol-1,4-dion

400 mg (1,14 mmol) 3,6-Bis(2'-methoxyphenyl)-2,5-dihydropyrrolo-(3,4-c)pyrrol-1,4-dion und 2,08 g (11,2 mmol) p-Toluolsulfonsäuremethylester werden mit 1,2 g Kaliumcarbonat in 5 ml DMF analog zu den Angaben in Chem. Ber. 120, 1075-1078 (1987) umgesetzt und aufgearbeitet. Ausb. 360 mg (84 %) orangefarbene Kristalle (IIIb). Umwandlungspunkt 195°C, Schmp. 281-282°C (aus Ethanol/ Chloroform 3:1). R_{f} (tert-Butylmethylether) = 0,44.
- UV(CHCl₃):: λₘₐₓ(lg ∈) = 454 nm(4,206).
- Fluoreszenz (CHCl₃):: λₘₐₓ = 514 nm.
- Fluoreszenzquantenausb.:: 0̸(CHCl₃) = 0,68,
0̸(CH₃CN) = 0,59.

| | | | |
|---|---|---|---|
| IIIa: C₂₂H₂₀N₂O₄ (376,4) | Ber. C 70,20 | H 5,35 | N 7,44 |
| | Gef. C 69,90 | H 5,31 | N 7,47 |
| IIIb: C₂₂H₂₀N₂O₄ (376, 4) | Ber. C 70,20 | H 5,35 | N 7,44 |
| | Gef. C 70,17 | H 5,29 | N 7,46 |

Das als Ausgangsprodukt benötigte 3,6-Bis(2'-methoxyphenyl)-2,5-dihydropyrrolo-(3,4-c)pyrrol-1,4-dion kann wie folgt hergestellt werden:

### 2-Methoxyacetophenon

Zu einer Mischung aus 130 g Kaliumhydroxyd in 1,1 1 Wasser und 260 g (1,90 mol) 2-Hydroxyacetophenon werden unter Kühlung und starkem Rühren 250 g (1,98 mol) Dimethylsulfat so zugetropft, daß sich die Reaktionslösung nicht über 40°C erwärmt. Nachdem anschließend für 30 Minuten die Temperatur auf 80°C gehalten wird, läßt man auf Raumtemperatur abkühlen und schüttelt mit insgesamt 250 ml Chlorform aus. Die organische Phase wird dreimal mit je 100 ml 2n Natronlauge geschüttelt und dann über Natriumsulfat getrocknet. Nach Filtration und Abrotieren des Lösungsmittels destilliert man das erhaltene Öl im Vakuum.

Ausbeute 223,7 g (78,4 %) farblose Flüssigkeit, Sdp. 121-124°C/21,28 mbar.

### 2-Methoxy-benzoylessigsäuremethylester

500 ml Dimethylcarbonat werden mit 58,5 g (1,08 mol) Natriummethanolat versetzt. Es wird dann bis zum Sieden erhitzt, und dabei werden Spuren von Methanol abdestilliert. Unter kräftigem Rühren läßt man bei 86°C während 4,5 Stunden 153,5 g (1,02 mol) 2-Methoxyacetophenon zutropfen. (Wird die Reaktionsmischung zu zähflüssig, empfiehlt sich eine weitere Zugabe von Dimethylcarbonat.) Anschließend läßt man noch 30 Minuten nachrühren und destilliert das Lösungsmittel unter vermindertem Druck ab. Nach Abkühlung auf Raumtemperatur wird mit 350 ml Wasser und 70 ml Eisessig versetzt und über Nacht gerührt. Das gebildete Öl trennt man ab und schüttelt die wässrige Phase mit insgesamt 300 ml Essigester aus. Die vereinigten organischen Phasen werden zuerst mit einer 6 %igen Natriumhydrogencarbonat-Lösung (100 ml), dann mit einer Natriumchlorid-Lösung gewaschen (das Natriumchlorid fördert die Phasentrennung). Nach Trocknen der Essigester-Phase über Natriumsulfat, Filtration und nach Abrotieren des Lösungsmittels wird das zurückbleibende Öl im Hochvakuum destilliert.

Ausbeute 92,7 g (43 %) gelbliches Öl, Sdp. 118-119°C/0,01 mbar, n_{D} ²⁰ = 1,5401.

### 2-Methoxy-benzoylbernsteinsäuredimethylester

91,16 g (440 mmol) 2-Methoxy-benzoylessigsäuremethylester, 57,5 g (530 mmol) Chloressigsäuremethylester, 280 ml Aceton, 190 ml Dimethoxyethan und 70,0 g (510 mmol) Kaliumcarbonat werden für 22 Stunden unter Rückfluß gekocht. Nach Abkühlen auf Raumtemperatur wird abgenutscht und der Rückstand mit n-Hexan gewaschen. Am Rotationsverdampfer engt man die vereinigten Filtrate ein und versetzt das zurückbleibende Öl mit 70 ml Methanol. Nach kurzer Zeit kristallisiert der 2-Methoxy-benzoylbernsteinsäuredimethylester aus.
Ausbeute 73,7 g (59,0 %) farblose Kristalle, Schmp.
75-76°C (Methanol), R_{f} (Kieselgel/Essigester) = 0,83.

| | | |
|---|---|---|
| C₁₄H₁₆O₆ (280,2) | Ber. C 59,99 | H 5,75 |
| | Gef. C 60,28 | H 5,75 |

### 3-Methoxycarbonyl-2-(2'-methoxyphenyl) -2-pyrrolin-5-on

152,5 g (1,98 mol) Ammoniumacetat und 50,0 g (0,18 mol) 2-Methoxy-benzoylbernsteinsäuredimethylester werden in 175 ml Eisessig) zwei Stunden unter Rückfluß gekocht (Ölbad 120-130°C). Die abgekühlte dunkelgrüne Reaktionsmischung läßt man in ca. 500 ml Eiswasser unter Rühren eintropfen. Nach Abnutschen und Trocknen über Calciumchlorid erhält man ein maigrünes Pulver (Schmp. 163-167°C), das direkt für die Folgereaktion eingesetzt wird.
Ausbeute 37,4 g (84 %), Schmp. 165-166°C (aus Methanol),
R_{f} (Kieselgel/Essigester) = 0,74.

| | | | |
|---|---|---|---|
| C₁₃H₁₃NO₄ (247,2) | Ber. C 63,15 | H 5,67 | N 5,67 |
| | Gef. C 63,45 | H 5,42 | N 5,41 |

### 3,6-Bis(2'-methoxyphenyl)-2,5-dihydropyrrolo(3,4-c)-pyrrol-1,4-dion

12,0 g (48,6 mmol) 3-Methoxycarbonyl-2-(2'-methoxyphenyl)-2-pyrrolin-5-on, 28,0 g (249 mmol) Kalium-tert-butylat und 14,0 g (105 mmol) 2-Methoxybenzonitril werden in 100 ml tert-Amylalkohol unter Stickstoff und ständigem Rühren drei Tage unter schwachem Rückfluß gehalten. Nach Abkühlen auf ca. 60°C versetzt man zuerst mit 100 ml Methanol und dann mit 20 ml Eisessig. Das ausgefallene Produkt wird über eine Glasfritte (G4) abgenutscht und mehrmals mit Methanol gewaschen.
Ausbeute 11,89 g (70,2 %) dunkelrotes Pulver.
Schmp. 336-337°C Zers. (aus Toluol).
R_{f} (Kieselgel-Toluol/Aceton = 4:1).
UV (DMF): λₘₐₓ (lg ∈) = 525 nm (4.496), 489 nm (4,397), 460 nm (sh).
Fluoreszenz (DMF): λₘₐₓ = 533 nm, 575 nm.

| | | | |
|---|---|---|---|
| C₂₀H₁₆N₂O₄ (348,3) | Ber. C 68,95 | H 4,62 | N 8,04 |
| | Gef. C 68,47 | H 4,51 | N 8,24 |

alternatives Herstellungsverfahren:
12,51 g (93,95 mmol) 2-Methoxybenzonitril, 10,75 g Kalium-tert-butylat und 9,57 g (46,8 mmol) Bernsteinsäurediisopropylester werden analog zu den Angaben in Chem. Ber. 120, 1075-1078 (1987) umgesetzt und aufgearbeitet.
Ausbeute 400 mg (2,4 %) dunkelrot schillernde Kristalle.
Schmp. 336-337°C Zers. (aus Toluol).

### Beispiel 2

### 3,6-Bis(2'-methoxypenyl)-2,5-dihydro-2,5-dimethylpyrrolo(3,4-c)pyrrol-1,4-dion

Eine Mischung aus 500 mg (1,5 mmol) 3,6-Bis(2'-methoxyphenyl)-2,5-dihydropyrrolo(3,4-c)pyrrol-1,4-dion und 1,5 g KOH (27 mmol) in 25 ml DMF wird unter Rühren bei Raumtemperatur mit 570 mg Methyliodid (0,25 ml = 4,0 mmol) versetzt. Nach ca. 3 Minuten beginnt das Produkt aus der Reaktionslösung auszufallen, und nach weiteren 5 Minuten werden 60 ml Eiswasser zugegeben. Der orangegelbe Niederschlag wurde abgenutscht, mit Wasser gewaschen und getrocknet.
Ausbeute 420 mg (75 %), Schmp. 281-282°C
(Ethanol/Chloroform 3:1).

## Patentansprüche

1. Farbstoffe der allgemeinen Formel I 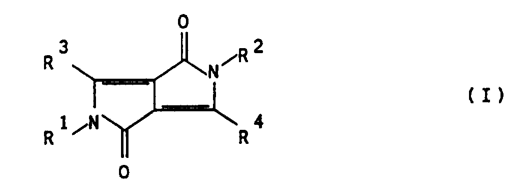 worin R¹ und R unabhängig voneinander (C₁-C₄)-Alkyl und R³ und R⁴ ortho-(C₁-C₄)-Alkoxyphenyl bedeuten.

2. Farbstoff gemäß Anspruch 1, dadurch gekennzeichnet, daß R¹ und R Methyl und R³ und R⁴ ortho-Methoxyphenyl bedeuten.

3. Verfahren zur Herstellung von Farbstoffen der allgemeinen Formel I gemäß Anspruch 1 und/oder 2, dadurch gekennzeichnet, daß eine Verbindung der allgemeinen Formel V 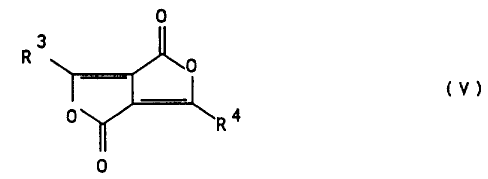 mit einer Verbindung der allgemeinen Formel R¹NH₂ oder einem Verbindungsgemisch von R¹NH₂/RNH₂ umgesetzt wird und gegebenenfalls zur Reinigung der Verbindung der Formel I eine extraktive Umkristallisation erfolgt, oder daß eine Verbindung der allgemeinen Formel VI 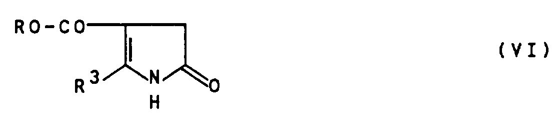 worin R z.B. einen niederen Alkylrest bedeutet und R³ die bereits genannte Bedeutung besitzt, mit einem Nitril der Formel R⁴CN, worin R⁴ die bereits genannte Bedeutung besitzt, umgesetzt wird.

4. Verwendung von Farbstoffen der allgemeinen Formel I gemäß Anspruch 1 und/oder 2 als fluoreszierende Pigmente.

5. Verwendung von Farbstoffen der allgemeinen Formel I gemäß Anspruch 1 und/oder 2 als Speichermedien in optischen Datenspeichern.

6. Adressierbarer und optisch auslesbarer Datenspeicher, dadurch gekennzeichnet, daß er als Speichermedium einen Farbstoff der allgemeinen Formel I gemäß Anspruch 1 und/oder 2 enthält.

7. Verfahren zum Einlesen und/oder Auslesen von Daten in einen Datenspeicher nach Anspruch 6, dadurch gekennzeichnet, daß die Informationen durch Zufuhr von Energie eingelesen werden, wodurch eine Modifikation des Farbstoffs in eine anders gefärbte und anders fluoreszierende Modifikation umgewandelt wird und/oder daß die verschiedene Absorption des in verschieden gefärbten Modifikationen vorliegenden Speichermediums als Kriterium für den Auslesevorgang und/oder daß die verschiedene Fluoreszenz des in verschieden fluoreszierenden Modifikationen vorliegenden Speichermediums als Kriterium für den Auslesevorgang benutzt wird.

8. Verfahren zum Löschen von Daten in einem Datenspeicher gemäß Anspruch 6, dadurch gekennzeichnet, daß das Speichermedium durch Zufuhr von Energie geschmolzen oder in der Schmelze einer Hilfssubstanz gelöst wird und aus der Schmelze oder Lösung in einer thermodynamisch metastabilen Modifikation auskristallisiert.

## Claims

1. Dyes of the general formula I 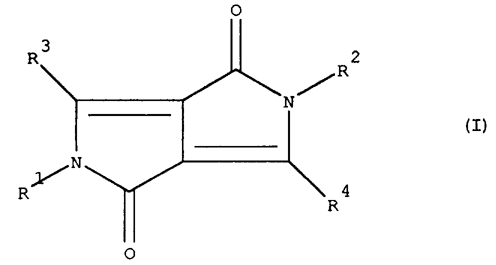 wherein R¹ and R independently of one another denote (C₁-C₄)-alkyl and R³ and R⁴ denote ortho-(C₁-C₄)-alkoxyphenyl.

2. Dye according to Claim 1, characterized in that R¹ and R denote methyl and R³ and R⁴ denote ortho-methoxyphenyl.

3. Process for preparing dyes of the general formula I according to Claim 1 and/or 2, characterized in that a compound of the general formula V 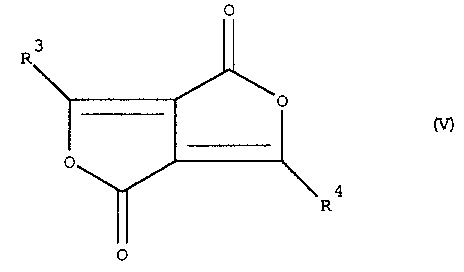 is reacted with a compound of the general formula R¹NH₂ or a mixture of compounds of R¹NH₂ / RNH₂ and that, optionally, an extractive recrystallization is effected to purify the compound of Claim 1, or that a compound of the general formula VI 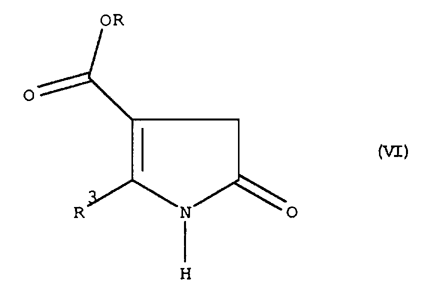 wherein R denotes for example a lower alkyl radical and R³ has the meaning indicated, is reacted with a nitrile of the formula R⁴CN wherein R⁴ has the meaning indicated.

4. Use of dyes of the general formula I according to Claim 1 and/or 2 as fluorescent pigments.

5. Use of dyes of the general formula I according to Claim 1 and/or 2 as storage media in optical data memories.

6. Addressable and optically readable data memory characterized in that it contains as a storage medium a dye of the general formula I according to Claim 1 and/or 2.

7. Process for entering data into and/or reading data from a memory according to Claim 6, characterized in that the information is entered by supplying energy, as a result of which a modification of the dye is transformed into a differently colored or differently fluorescing modification and/or that the different absorption of the storage medium present in differently colored modifications is used as a criterion for the reading process and/or that the different fluorescence of the storage medium present in differently fluorescent modifications is used as criterion.

8. Process for erasing data in a data memory according to Claim 6, characterized in that the storage medium is melted by supplying energy or is dissolved in the melt of an auxiliary substance and crystallizes out of the melt or solution in a thermodynamically metastable modification.

## Revendications

1. Colorants de formule générale I 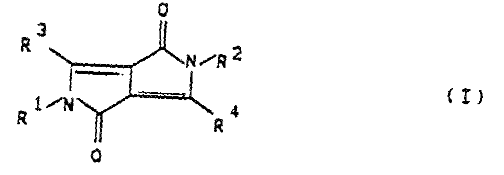 dans laquelle R¹ et R, indépendamment l'un de l'autre, représentent alkyle en C₁-C₄ et R³ et R⁴ représentent orthoalcoxyphényle en C₁-C₄.

2. Colorant selon la revendication 1, caractérisé en ce que R¹ et R représentent méthyle et R³ et R⁴ représentent ortho-méthoxyphényle.

3. Procédé pour la préparation de colorants de formule générale I selon la revendication 1 et/ou 2, caractérisé en ce qu'on fait réagir un composé de formule générale V 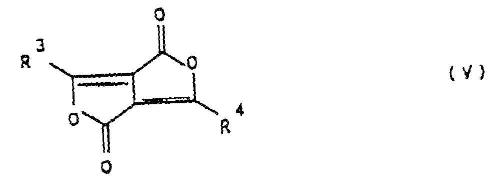 avec un composé de formule générale R¹NH₂ ou un mélange de composés R¹NH₂/RNH₂ et éventuellement, pour purifier le composé de formule I, on effectue une recristallisation extractive, ou en ce qu'on fait réagir un composé de formule générale VI 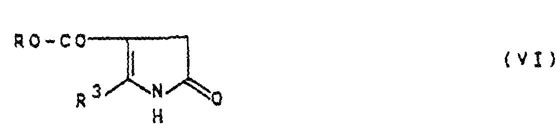 dans laquelle R représente par exemple un radical alkyle inférieur et R³ a la signification déjà connue, avec un nitrile de formule R⁴CN dans laquelle R⁴ a la signification déjà connue.

4. Utilisation de colorants de formule générale I selon la revendication 1 et/ou 2 comme pigments fluorescents.

5. Utilisation de colorants de formule générale I selon la revendication 1 et/ou 2 comme support d'enregistrement dans des mémoires optiques d'informations.

6. Mémoires d'informations adressables et optiquement extractibles, caractérisées en ce que le support d'enregistrement contient un colorant de formule générale I selon la revendication 1 et/ou 2.

7. Procédé pour l'enregistrement et/ou l'extraction d'informations dans une mémoire d'informations selon la revendication 6, caractérisé en ce que les informations sont enregistrées au moyen de l'apport d'énergie, ce qui provoque un changement du colorant en une modification colorée différemment et différemment fluorescente et/ou en ce qu'on utilise l'absorption différente du support d'informations présent dans les modifications différemment colorées comme critère pour le procédé d'extraction et/ou en ce qu'on utilise la fluorescence différente du support d'information présent dans les différentes modifications fluorescentes comme critère pour le processus d'extraction.

8. Procédé pour effacer les informations dans un support d'informations selon la revendication 6, caractérisé en ce que le support d'information est fondu ou dissout dans une substance auxiliaire fondue au moyen de l'apport d'énergie et cristallise à partir de la fonte ou solution sous une forme thermodynamiquement métastable.
